# EUROPEAN PATENT SPECIFICATION

(11) **EP 1 088 081 B1**
(45) Date of publication and mention of the grant of the patent: **09.03.2005**
(21) Application number: 99930655.8
(22) Date of filing: 25.06.1999
(51) Int. Cl.: C12N 15/55, C12N 15/82, C12N 9/72, C12N 5/10, A01H 5/00, A01H 5/10

(54) **PRODUCTION OF UROKINASE IN PLANT-BASED EXPRESSION SYSTEMS**
HERSTELLUNG VON UROKINASE IN PFLANZLICHEN EXPRESSIONSSYSTEMEN
PRODUCTION D'UROKINASE DANS DES SYSTEMES D'EXPRESSION VEGETAUX

(30) Priority: 26.06.1998 US 90911 P
(43) Date of publication of application: 04.04.2001
(73) Proprietor: Croptech Development Corporation, Blacksburg, VA 24060 (US)
(72) Inventor: OISHI, Karen, K., Draper, VA 24324 (US); ZHOU, Da-Feng, Blacksburg, VA 24060 (US)
(74) Representative: Baldock, Sharon Claire
(86) International application number: PCT/US1999/014292
(87) International publication number: WO 2000/000624

(56) References cited:
- EP-A- 0 620 279
- WO-A-97/10353
- DD-A- 263 081
- US-A- 5 723 755
- BECKER, CLAUDIA (1) ET AL: "Synthesis and accumulation of human tissue-type plasminogen activator (h- tPA ) in transgenic tobacco seeds" SHEWRY, P. R. [EDITOR];STOBART, K. [EDITOR]. PROCEEDINGS OF THE PHYTOCHEMICAL SOCIETY OF EUROPE, (1993) VOL. 35, PP. 325-331. PROCEEDINGS OF THE PHYTOCHEMICAL SOCIETY OF EUROPE; SEED STORAGE COMPOUNDS: BIOSYNTHESIS, INTERACTIONS, AND MANIPULATION. PU, XP000856303 cited in the application
- ROUF S A ET AL: "Tissue-type plasminogen activator: characteristics, applications and production technology" BIOTECHNOLOGY ADVANCES,GB,ELSEVIER PUBLISHING, BARKING, vol. 14, no. 3, page 239-266 XP004046107 ISSN: 0734-9750

## Description

### FIELD OF THE INVENTION

The present invention relates to the production of human and animal urokinase ("UK") by expressing the genetic coding sequence of a human or animal urokinase in a plant expression system. The plant expression system provides for post-translational modification and processing to produce biologically active UK.

The invention is demonstrated herein by working examples in which transgenic tobacco plants produce human urokinase which is biologically active. The recombinant urokinase produced in accordance with the invention may be used for a variety of purposes including but not limited to thrombolytic therapy and non-therapeutic uses such as clearance of surgical catheters.

### BACKGROUND OF THE INVENTION

### Thrombolytic agents

Acute myocardial infarction (heart attacks) is one of the major causes of mortality in the United States (Anderson & Willerson, *N. Engl.* J. *Med.* 329:703-709 (1993)). In the majority of cases, a heart attack is caused by an obstruction of the coronary artery by the formation of a clot site (thrombosis). Administration of thrombolytic drugs such as streptokinase, UK and tPA can significantly decrease the incidence of early mortality. Of the thrombolytic drugs available, only UK and tPA have activity that is specific to the site of clotting. Recombinant and non-recombinant forms of t-PA and UK have been successfully used as thrombolytic agents in humans suffering strokes and heart attacks. (Gurewich, N. Engl. *J. Med.* 330:291 (1993).

The biological process of formation and dissolution of blood clots is regulated by a complex coordinated reaction between the blood coagulation cascade and the endogenous fibrinolytic pathway (Gurewich et al., Ann. *N.Y. Acad. Sci.* 4:224-232 (1992)). The process of formation of thrombosis occurs when there is an endothelial disruption exposing the subepiderminal surface. Glycoprotein receptors on the surface of circulating platelets attach to the subepiderminal surface. The formation of the platelet plug, crosslinked platelets, is mediated by von Willebrand factor and fibrinogen. The blood coagulating factor, Factor XIII, catalyzes the cross-linking of the fibrin strands to stabilize the platelet plug (Anderson *et al., N. Engl J. Med.* 329:703 (1993)). The fibrinolytic process, known as thrombolysis, is regulated by activating the circulating zymogen plasminogen with either extrinsic tissue-type plasminogen activator (t-PA) or constitutive intrinsic urokinase-type plasminogen activator (UK) (see TABLE 1). Plasminogen activators convert inactive zymogen plasminogen to plasmin, an active serine protease, which functions to control extracellular clot lysis by degrading fibrin. UK specifically binds to platelet associated at the site of thrombosis and tPA specifically binds to fibrin. Mice lacking the UK gene display an immediate accumulation of intravascular plasma clots. By contrast, tPA-deficient mice failed to display the same symptoms. Administration of UK to the UK deficient mice induced lysis of the plasma clots. (Carmeliet *et al*., *Nature,* 368:419 (1994)). In addition, the platelet binding UK has been shown to be primarily responsible for the exceptionally high endogenous fibrinolytic activity in dogs (Lang *et al*., *Circulation* 87:1990 (1993)). These results indicate that UK plays a primary role in the fibrinolytic process in mammals.

**TABLE 1.**

| **Fibrinolysis ("Clot Busting")** | |
|---|---|
| Plasma clot formation initiates both extrinsic and intrinsic fibrinolysis: | |
| Extrinsic Pathway | Intrinsic Pathway |
| • Mediated by tPA | • Mediated by UK, Factor XII, kallikrein and platelets |
| • tPA is triggered to be secreted by endothelium when clots form | • UK constitutively circulates in plasma |
| • tPA binds fibrin | • Fibrin specificity |
| • Fibrin clot binding inhibited by degraded fibrin | • Platelet clot binding and activation by platelet surface bound kallikrein. |
| | • 20% of plasma UK is associated with platelets |
| • Moderate catalytic efficiency of clot lysis (higher doses required than with UK) | • Maximum rate of clot lysis is two times faster than tPA |
| • Both bound and unbound tPA has a very short half-life | **•** Unbound UK has a half-life of 7 min. Bound UK has a longer half-life |

### Urokinase

The proenzyme of UK is a glycoprotein having a molecular weight of 47-56 kDa (Husain *et al*., *Arch. Biochem: Biophys.* 220:31 (1983)). This protein contains a single polypeptide chain with four functionally defined domains: (i) the N-terminal domain, which has homology to epidermal growth factor (EGF); (ii) the "kringle" domain; (iii) the connecting peptide domain; and (iv) the serine protease domain. The inactive single chain prourokinase is cleaved at Lys¹⁵⁸-Ile¹⁵⁹ by clot-localized plasmin into the two-chain (20 and 30 kD) active High Molecular Weight urokinase (HMW UK), which in turn activates plasminogen on the thrombus surface (Stoppelli *et al., Proc. Nat'l* Acad. Sci. USA, 82:4939 (1985); Appella et al., J. Biol. Chem. 262:4437)). In addition to the two-chain 47-56 kDa HMW UK, a 33 kDa active Low Molecular Weight (LMW) UK isoform has been used as a therapeutic agent (Stoppelli *et al.*, *J. Biol.* Chem. 262:4437 (1985)). Human UK has a single N-linked carbohydrate moiety at Asn-302 (within the serine protease domain) which may be required for specificity in serum, but is not required for activity or stability in serum (Hoylaerts *et al.*, *J. Biol.Chem.* 257:2912 (1982); Melnick *et al.*, *J. Biol. Chem.* 265:801 (1990)) . The carbohydrate group found at Asn-302 is both complex and heterogenous. Human UK also has 12 disulfide linkages and is phosphorylated (Holmes *et al.*, *Bio*/*Tech.* 3:923 (1985)).

UK protein has been isolated from human urine, kidney cells (Verde *et al.*, *Proc. Nat'l. Acad. Sci. USA* 81:4727 (1984)), plasma, carcinoma cell lines (Detroit 562 cells, Holmes et al., Biotechnology, 3:923 (1985)), A431 cells), and normal fibroblast cells (Cheng et al., Gene 69:357 (1988)). Genomic DNA and cDNA encoding UK has been isolated from human cells and other mammals. (Jacobs *et al.*, *DNA* 4:139 (1985); Holmes *et al.*, *supra;* Verde *et al.*, *Proc. Nat'l Acad. Sci. USA* 81:4727 (1984); Nagai et al., Gene 36:183 (1985)). Outside the United States, human urine is the primary commercial source of UK. Because of safety concerns about use of urine as a protein source, UK has been produced in the United States chiefly via mammalian'tissue culture methods (Cheng et al., Gene 69:357 (1988)). UK also is secreted from various cell types in addition to kidney and is found in plasma at a similar concentration to tPA (2-4 ng/ml) in humans.

Recombinant preUK has been expressed in both nonglycosylated and glycosylated forms, and has been found to be activated by plasmin. Nonglycosylated UK has been expressed in *E. coli* (Jacobs *et al., DNA* 4:139 (1985)); Holmes *et al., Bio*/*Technology* 3:923 (1985)) and yeast (Zarowski et al., 1989; Melnick *et al.*, *J. Biol.* Chem. 265:801 (1990)). The majority of UK produced in E. coli consisted inactive unfolded protein associated with inclusion bodies (Winker *et al. Biochem.* 25:4041 (1986)). The highest activity obtained for recombinant UK was from a baculovirus expression system in insect cells, where 90% of the secreted UK protein was found to be single-chain UK activated by plasmin (King *et al.*, *Gene* 106:151 (1991)). Finally, although UK has been isolated from transgenic animal milk, this system suffers from the disadvantage that endogenous UK is present in high levels in milk, hindering purification of the human UK (Deharveng *et al., J. Dairy Sci.* 74:2060 (1991)).

To date UK has not been expressed in any plant system. Attempts have been made, however, to express tPA in tobacco seeds. Using a seed-specific legumin promoter, tPA and the signal peptide-less tPA were expressed in transgenic tobacco. However, no enzymatic activity was detected with or without plasmin treatment in seeds expressing either construct (Becker *et al*., *Ann. Proc. Cytochem. Soc. of Eur.* 35:325-331 (1993)). Western blot analysis under non-reducing conditions using anti-tPA antibodies indicated the presence of a 66-69 kD protein that comigrated with human tPA. Western analysis under reducing conditions detected a 19 kD protein, much smaller than would have been expected from correctly cleaved human tPA. This was an indication that tPA polypeptide was produced in seeds but not in a form which could be cleaved by plasmin into an active enzyme.

EP-A-0 620 279 discloses novel derivatives of the human protein urokinase, together with methods of making them in bacteria, yeast and animal cell culture using recombinant DNA technology.

WO 97/10353 discloses the production of enzymatically active recombinant human and animal lysosomal enzymes involving construction and expression of recombinant expression constructs comprising coding sequence of human or animal lysosomal enzymes in a plant expression system. Transgenic tobacco plants having recombinant expression constructs comprising human hGC and IDUA nucleotide sequences were shown to produce enzymatically active modified human glucocerebrosidae and human α-L-iduronidase.

Overall, the production of UK in mammalian cells and yeast has been found to be inefficient (Hiramatsu *et al*., Gene 99:235 (1991)). It is apparent, therefore, that methods of producing UK that are inexpensive, reliable, and amenable to large scale production are greatly to be desired. In particular, methods for producing urokinase in plants that produce correctly processed, biologically active, protein are highly desirable.

### SUMMARY OF THE INVENTION

It is therefore an object of the present invention to provide methods for producing biologically active urokinase in plants.

It is also an object of the present invention to provide products, including expression constructs, DNA vectors, and transgenic plants, that are particularly adapted to implementing such methods.

It is a further object of the present invention to transiently express a gene encoding UK in transformed plant cells.

It is a further object of the invention to provide recombinant urokinase products prepared by such methods.

In accomplishing these objects, there has been provided, in accordance with one aspect of the present invention, a method for producing a biologically active urokinase in a transgenic plant, comprising the steps of growing the transgenic plant containing a recombinant expression construct encoding the urokinase and a promoter that regulates expression of the nucleotide sequence, so that the urokinase is expressed by the transgenic plant, and recovering the urokinase from an organ of the transgenic plant. The organ may be a leaf, stem, root, flower, fruit or seed.

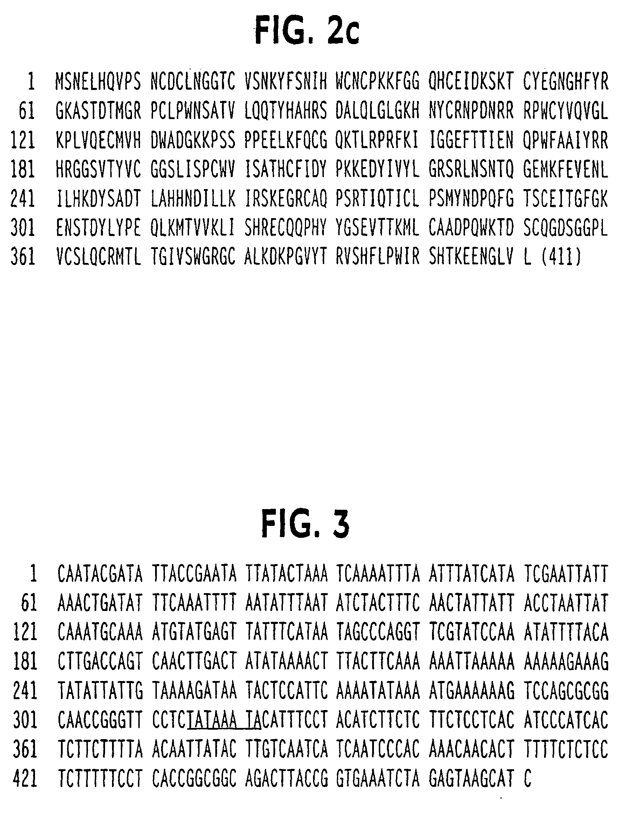

In one embodiment, the promoter is an inducible promoter, which may be induced before or after the transgenic plant is harvested. In a preferred embodiment, the inducible promoter may be induced by mechanical gene activation. In another preferred embodiment, the inducible promoter comprises the nucleotide sequence shown in Figure 3. In another embodiment, the transgenic plant is a transgenic tobacco plant.

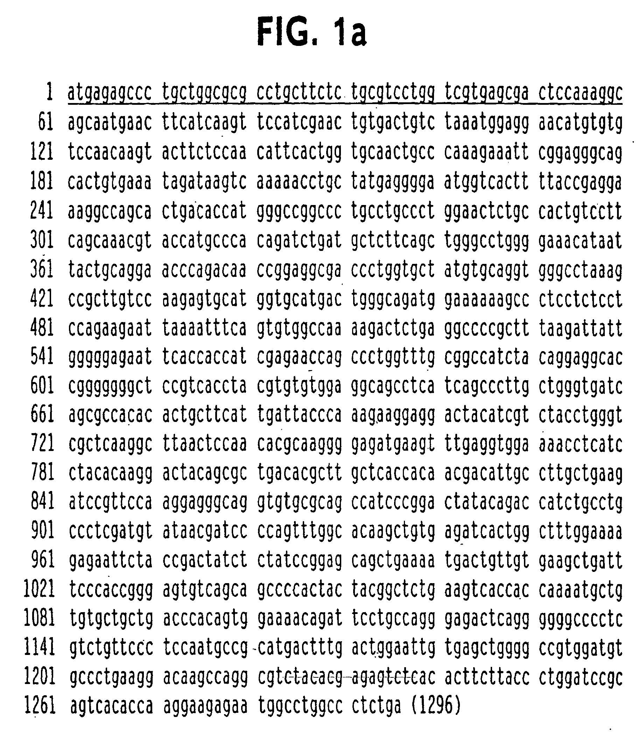

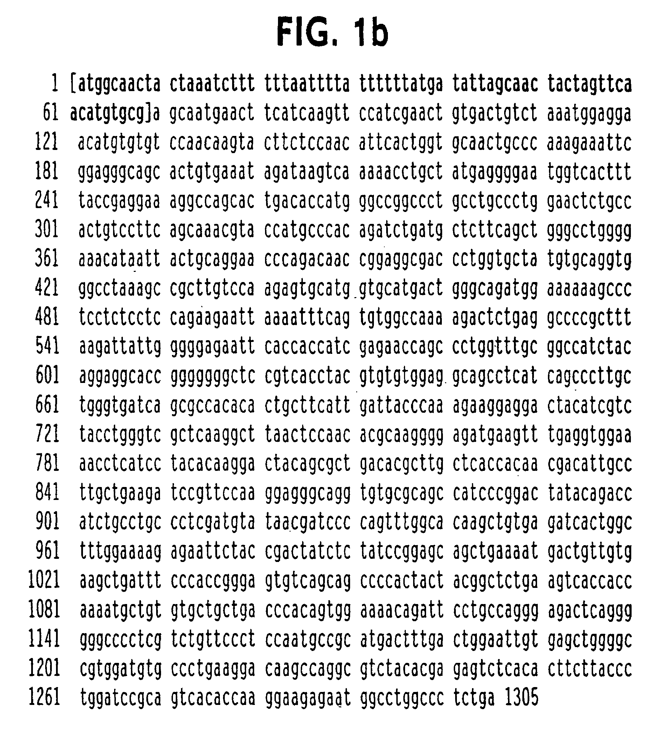

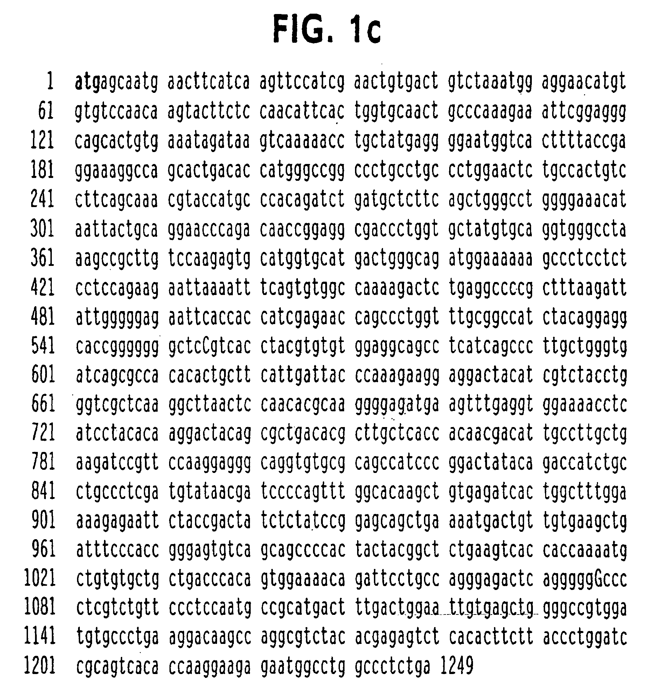

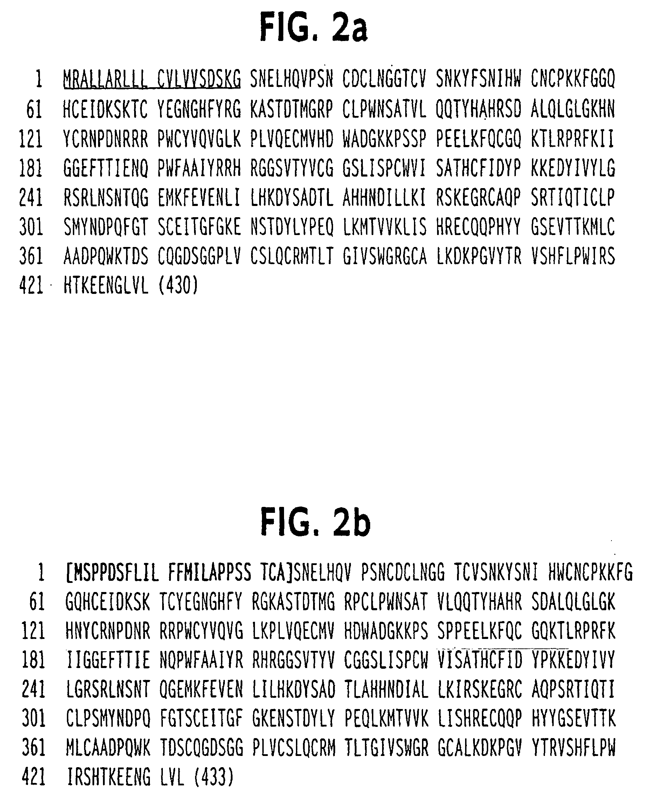

In yet another embodiment the urokinase is a human urokinase. In a preferred embodiment, the urokinase comprises amino acids 2-411 of the sequence shown in Figure 2c. In still another embodiment, the expression construct encodes the amino acid sequence shown in Figure 2a, 2b, or 2c. In another embodiment, the expression construct comprises the nucleotide sequence shown in Figure 1a, 1b, or 1c. In a still further embodiment, the expression construct comprises pCT92, pCT97, or pCT111. In another embodiment, the nucleotide sequence encodes preprourokinase or modified preprourokinase.

In accordance with another aspect of the invention, there has been provided a recombinant expression construct comprising a nucleotide sequence encoding a urokinase and a promoter that regulates the expression of the nucleotide sequence in a plant cell. In one embodiment, the promoter is an inducible promoter. In a preferred embodiment the inducible promoter may be induced by mechanical gene activation. In another embodiment, the inducible promoter comprises the nucleotide sequence shown in Figure 3. In still another embodiment, the urokinase is a human urokinase. In yet another embodiment, the expression construct is contained in a plant transformation vector. In a further embodiment, the expression construct is contained within a plant cell, tissue or organ.

In accordance with yet another aspect of the invention, there has been provided a transgenic plant, plant cell, or part of a plant capable of producing an biologically active urokinase, where the transgenic plant or plant cell has a recombinant expression construct comprising a nucleotide sequence encoding a urokinase or modified urokinase and a promoter that regulates expression of the nucleotide sequence in the transgenic plant or plant cell. In one embodiment, the promoter is an inducible promoter, and in a preferred embodiment may be induced by mechanical gene activation. In another preferred embodiment, the inducible promoter comprises the nucleotide sequence shown in Figure 3. In still another embodiment, the transgenic plant or plant cell is a transgenic tobacco plant or tobacco cell, and in another embodiment, the urokinase is a human urokinase. In a further embodiment, the plant, plant cell, or part of a plant is a leaf, stem, root, flower or seed.

In accordance with yet another aspect of the invention, there has been provided a urokinase that is biologically active and that is produced according to a process comprising growing a transgenic plant containing a recombinant expression construct comprising a nucleotide sequence encoding the urokinase and a promoter that regulates expression of the nucleotide sequence so that the urokinase is expressed by the transgenic plant; and recovering the urokinase from an organ of the transgenic plant. The organ may be a leaf, stem, root, flower, fruit or seed. In a preferred embodiment, the promoter is an inducible promoter, which may be induced before or after the transgenic plant is harvested. In a preferred embodiment, the inducible promoter comprises the nucleotide sequence shown in Figure 3. In another embodiment, the transgenic plant is a transgenic tobacco plant, and in another embodiment, the urokinase is a human urokinase.

Other objects, features and advantages of the present invention will become apparent from the following detailed description. It should be understood, however, that the detailed description and the specific examples, while indicating preferred embodiments of the invention, are given by way of illustration only, since various changes and modifications within the spirit and scope of the invention will become apparent to those skilled in the art from this detailed description.

### BRIEF DESCRIPTION OF THE FIGURES

**Figure 1a** shows the nucleotide sequence of the human preprourokinase coding region of CT92. The underlined nucleotide sequences (nucleotides 1-59) represent the human signal peptide of human preprourokinase protein, and the non-underlined sequence is urokinase (EC 3.4.99.26). The sequences in bold indicate differences between the published nucleotide sequence and the CT92 coding sequence. Nucleotide #1289 has been found as t or c (GenBank Accession D00244).
**Figure 1b** shows the nucleotide sequence of the coding region of CT111, containing the potato patatin signal peptide and the human prourokinase sequence. The human prourokinase nucleotide sequence (nucleotides 69-1305) has homology to the sequence described by Jacobs *et al. DNA* 4:139-146 (1985), GENBANK ACCESSION NO. :X02760 M10113. The bracketed sequence (nucleotides 1-69) is the signal peptide of potato patatin protein (Iturriaga *et al.*, *Plant Cell* 1:381-390 (1989)(GenBank Accession No. M21878). The bolded sequences indicate differences between the published nucleotide sequence of preprourokinase and the CT111 coding sequence.
**Figure 1c** shows the nucleotide sequence of the coding region of CT97 containing the human prourokinase sequence (with no signal peptide sequence) . The bolded sequences are differences between the published nucleotide sequence of prourokinase (Jacobs et al., supra) and the CT97 coding sequence:
**Figure 2a** shows the deduced amino acid sequence of CT92. The underlined sequence represents the human signal peptide. The non-underlined region codes for human urokinase (EC 3.4.99.26). Amino acid #430 has been found as an A or V in human preUK sequences (Verde et *al*., *supra).*
**Figure 2b** shows the deduced amino acid sequence of CT111. The boxed bolded sequence is the potato patatin signal peptide and represents changes to the amino acid sequence of human preprourokinase. The non-boxed region encodes human urokinase (EC 3.4.99.26).
**Figure 2c** shows the deduced amino acid sequence of CT97. The bolded amino acid represents changes to the published human urokinase (EC 3.4.99.26).
**Figure 3** shows the nucleotide sequence of the MeGA promoter.

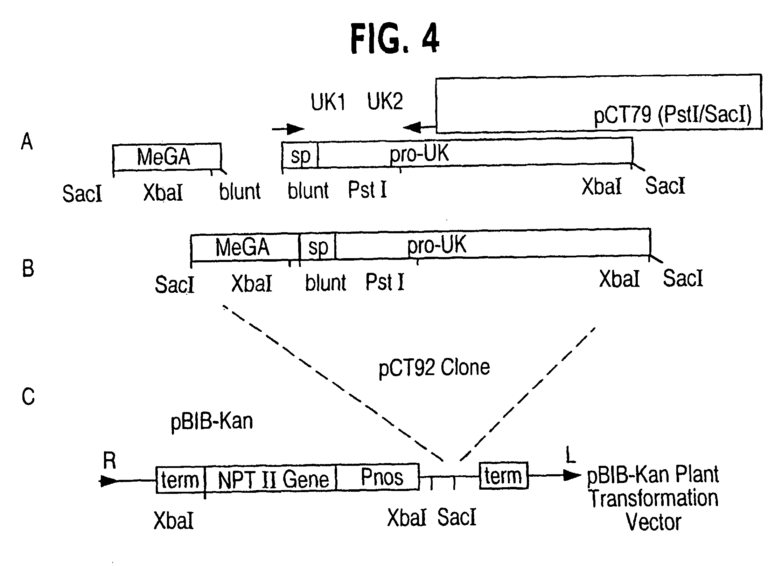

**Figure 4** shows a schematic representation of the strategy for cloning MeGA:preUK into the binary vector pBiB-Kan. R and L represent T-DNA right and left borders which precisely demarcate the DNA inserted into the plant genome. NPTII is the kanamycin selectable marker, term is the polyadenylation/terminator signal and Pnos is a promoter from *Agrobacterium tumefaciens* nopaline synthetase gene. UK1 and UK2 are the oligonucleotide primers designated in TABLE 2. The human signal peptide is designated by "sp". The coding region of urokinase is designated by sp-preUK. Restriction endonuclease sites that were digested with mung bean nuclease are designated as "blunt".
**Figure 5** shows a schematic representation of the cloning strategy of the MeGA:patatin signal peptide/UK into the binary vector pBiB-Kan. R and L, NPTII, term, and Pnos are as defined above for Figure 4. UK3 and UK2 are the oligonucleotide primers designated in TABLE 2. The human signal peptide and patatin signal peptide are designated by sp and psp, respectively. The coding region of UK lacking a signal peptide is designated by UK. Restriction endonuclease sites that are digested with mung bean nuclease are designated as "blunt."
**Figure 6** shows a schematic representation of the cloning strategy of MeGA:signal peptideless UK into the binary vector pBiB-Kan. UK3 and UK2 are the oligonucleotide primers designated in TABLE 2. The human signal peptide is designated by sp. The coding region of UK lacking the signal peptide is designated by UK. Restriction endonuclease sites digested with mung bean nuclease are designated by "blunt".
**Figure 7** shows the results of a fluorometric assay of total UK activity and the portion of activity secreted into the incubation buffer.
**Figure 8** shows the UK activity found in the incubation medium for CT92 plants following induction of protein production.
**Figure 9** shows the UK activity found in the cell extracts from CT97 plants following induction of protein production.
**Figure 10** shows the UK activity found in the incubation medium for CT111 plants following induction of protein production.

### DETAILED DESCRIPTION OF THE INVENTION

The present invention provides methods for producing human or animal urokinase ("UK") in transformed or transfected plants, plant cells or plant tissues, and involves constructing and expressing recombinant expression constructs comprising urokinase coding sequences in a plant expression system. The plant expression system provides appropriate co-translational and post-translational modifications of the nascent peptide required for processing, e.g., signal sequence cleavage, glycosylation, and sorting of the expression product so that a biologically active protein is produced. Using the methods described herein, recombinant urokinase is produced in plant expression systems from which the recombinant protein can be isolated and used for a variety of purposes.

The invention is exemplified by the genetic engineering of transgenic tobacco plants with three urokinase expression constructs. One construct comprises a nucleotide sequence encoding prepro-urokinase (containing the natural human signal sequence). Another construct comprises a nucleotide sequence encoding pro-urokinase fused to a sequence encoding a potato patatin signal peptide. The third construct comprises a nucleotide sequence encoding pro-urokinase lacking any signal sequence. Transgenic tobacco plants having the expression constructs produce urokinase that is biologically active.

The plant expression systems and the recombinant urokinase produced therewith have a variety of uses, including but not limited to: (1) the production of enzymatically active urokinase for the treatment of thrombolytic disorders and for clearing catheters; (2) the production of antibodies against urokinase for medical diagnostic use; (3) use in any commercial process that involves substrate hydrolysis, and (4) the production of modified proteins or peptide fragments to serve as precursors or substrates for further in vivo or *in vitro* processing to a specialized industrial form for research or therapeutic uses, such as to produce a therapeutic protein with increased efficacy or altered substrate specificity. These plant-expressed recombinant urokinase products need not be biologically active or identical in structure to the corresponding native animal or human urokinases in order to be useful for research or industrial applications.

The methods of the invention involve: (1) construction of recombinant expxession constructs comprising urokinase coding sequences and transformation vectors containing the expression constructs; (2) transforming or transfecting plant cells, plant tissues or plants with the transformation vectors; (3) expressing the urokinase coding sequences in the plant cell, plant tissue or plant; and (4) detecting and purifying expression products having urokinase activity.

The terms "urokinase" and "urokinase gene product," as used herein with respect to any such protein produced in a plant expression system, refer to a recombinant peptide expressed in a transgenic plant or plant cell from a nucleotide sequence encoding a human or animal urokinase, a modified human or animal urokinase, or a fragment, derivative or modification of such enzyme. Useful urokinases include but are not limited to single chain pro-urokinase, two-chain enzymatically active urokinase, and truncated enzymatically active (33 kD) urokinase. The skilled artisan will appreciate that other urokinase peptides are known and may be employed in the present invention. Useful modified human or animal urokinases include but are not limited to human or animal urokinases having one or several naturally-occurring or artificially-introduced amino acid additions, deletions and/or substitutions.

The term "urokinase coding sequence," as used herein, refers to a DNA or RNA sequence that encodes a protein or peptide, or a fragment, derivative or other modification thereof, which exhibits detectable enzymatic activity against a urokinase substrate, or that may be activated by proteolytic cleavage to exhibit detectable enzymatic activity against a urokinase substrate.

The term "biologically active" with respect to any recombinant urokinase produced in a plant expression system is used herein to mean that the recombinant urokinase is able to hydrolyze either the natural substrate, or an analogue or synthetic substrate thereof of the corresponding human or animal urokinase, at detectable levels.

The term "biologically active" is also used herein with respect to recombinant UK and modified UK produced in a plant expression system to mean that such UK molecules are (i) activated by plasmin cleavage, (ii) are able to hydrolyze plasminogen to plasmin, or (iii) that the UK can cleave the synthetic substrate at detectable levels, as described in more detail below.

The term "transformant" as used herein refers to a plant, plant cell or plant tissue to which a gene construct comprising a urokinase coding sequence has been introduced by a method other than transfection with an engineered virus.

The term "transfectant" refers to a plant, plant cell or plant tissue that has been infected with an engineered virus and stably maintains said virus in the infected cell.

Once a plant transformant or transfectant is identified that expresses a recombinant urokinase, one non-limiting embodiment of the invention involves the clonal expansion and use of that transformant or transfectant in the production and purification of biologically active recombinant urokinase. In another non-limiting embodiment of the invention, each new generation of progeny plants may be newly screened for the presence of nucleotide sequence coding for a urokinase, wherein such screening results in production by subsequent generations of plants of recoverable amounts of active recombinant urokinase, and wherefrom the enzyme is then purified.

The invention is divided into the following sections solely for the purpose of description: (a) genes or coding sequences for urokinase; (b) construction of recombinant gene constructs for expressing urokinase coding sequences in plant cell; (c) construction of plant transformation vectors comprising the expression constructs; (d) transformation/transfection of plants capable of translating and processing primary translation products in order to express an biologically active recombinant urokinase; (e) identification and purification of the recombinant urokinase so produced; (f) expansion of the number of transformed or transfected plants; and (g) methods of therapeutically using the recombinant urokinase.

### Genes or coding sequences for urokinases

The recombinant urokinases produced in accordance with this invention will have a variety of uses, probably the most significant being their use for thrombolytic therapy or for catheter clearance. Several cDNA sequences encoding urokinase have been described. (Jacobs *et al.*, *DNA* 4:139 (1985); Holmes *et al.*, Biotechnology, 3:923 (1985); Verde *et al., Proc. Nat'l Acad. Sci. USA* 81:4727 (1984); Nagai *et al.*, *Gene* 36:183 (1985)).

The nucleic acid sequences encoding urokinases which can be used in accordance with the invention include but are not limited to any nucleic acid sequence that encodes a urokinase, modified urokinase, or functional equivalent thereof, including but not limited to: (a) any nucleotide sequence that selectively hybridizes to the complement of a human or animal urokinase coding sequence under stringent conditions, e.g., washing in 0.1xSSC/0.1% SDS at 68°C (Ausubel *et al.*, eds., 1989, Current Protocols in Molecular Biology, Vol. I, Greene Publishing Associates, Inc. and John Wiley & Sons, Inc., New York, at page 2.10.3), and encodes a product homologous to the human or animal urokinase; and/or (b) any nucleotide sequence that hybridizes to the complement of the human or animal urokinase coding sequence under less stringent conditions, such as moderately stringent conditions, *e.g.*, washing in 0.2xSSC/0.1% SDS at 42°C (Ausubel *et al.*, 1989, *supra*), yet which still encodes a homologous gene product that is biologically active; and (c) any nucleotide coding sequence that otherwise encodes a protein from any organism capable of hydrolyzing a human or animal urokinase's plasminogen substrate or a substrate analogue.

The invention also includes but is not limited to: (a) DNA vectors that contain any of the foregoing nucleotide coding sequences and/or their complements; (b) DNA expression and transformation vectors that contain expression constructs comprising any of the foregoing nucleotide coding sequences operatively associated with a regulatory element that directs expression of the coding sequences in plant cells or plants; and (c) genetically engineered plant cells or plants that contain any of the foregoing coding sequences, operatively associated with a regulatory element that directs the expression of the coding and/or antisense sequences in the plant cell. As used herein, the term "regulatory element" includes but is not limited to inducible and non-inducible promoters, enhancers, operators and other elements known to those skilled in the art that drive and/or regulate gene expression. The invention also includes fragments, derivatives or other modifications of the DNA sequences described herein.

### Transformation vectors to direct the expression of urokinase coding sequences

### Urokinase expression constructs

In order to express a urokinase in a plant expression system, the urokinase coding sequence is inserted into an appropriate expression construct and the expression construct is incorporated into a transformation vector for transfer into cells of the plant. The expression construct is preferably constructed so that the urokinase coding sequence is operatively associated with one or more regulatory elements, including, *e.g.*, promoters and/or enhancers, necessary for transcription and translation of the urokinase coding sequence. Methods to construct the expression constructs and transformation vectors include standard in vitro genetic recombination and manipulation. See, for example, the techniques described in Weissbach and Weissbach, 1988, Methods For Plant Molecular Biology, Academic Press, Chapters 26-28.

Regulatory elements that may be used in the expression constructs include promoters which may be either heterologous or homologous to the plant cell. The promoter may be a plant promoter or a non-plant promoter which is capable of driving high levels transcription of a linked sequence in plant cells and plants. Nonlimiting examples of plant promoters that may be used effectively in practicing the invention include cauliflower mosaic virus (CaMV) 19S or 35S, *rbcS,* the promoter for the chlorophyll a/b binding protein, AdhI, NOS and HMG2, or modifications or derivatives thereof. The promoter may be either constitutive or inducible. For example, and not by way of limitation, an inducible promoter can be a promoter that promotes expression or increased expression of the urokinase nucleotide sequence after mechanical gene activation (MGA) of the plant, plant tissue or plant cell. One non-limiting example of such an MGA-inducible plant promoter is MeGA (described *infra*).

The expression constructs can be additionally modified according to methods known to those skilled in the art to enhance or optimize heterologous gene expression in plants and plant cells. Such modifications include but are not limited to mutating DNA regulatory elements to increase promoter strength or to alter the urokinase coding sequence itself. Other modifications include deleting intron.sequences or excess non-coding sequences from the 5' and/or 3' ends of the urokinase coding sequence in order to minimize sequence- or distance-associated negative effects on expression of urokinase, *e.g.,* by minimizing or eliminating message destabilizing sequences.

The expression constructs may be further modified according to methods known to those skilled in the art to add, remove, or otherwise modify peptide signal sequences to alter signal peptide cleavage or to increase or change the targeting of the expressed urokinase through the plant endomembrane system. For example, but not by way of limitation, the expression construct can be specifically engineered to target the urokinase for secretion, or vacuolar localization, or retention in the endoplasmic reticulum (ER).

In one embodiment, the expression construct can be engineered to incorporate a nucleotide sequence that encodes a signal targeting the urokinase to the plant vacuole. For example, and not by way of limitation, the N-terminal 143 amino acid domain derived from the plant vacuolar protein, proaleurain (Holwerda et al., 1992, *supra*; Holwerda *et al*., 1990, *supra),* may be engineered into the expression construct to produce a signal peptide-urokinase fusion product upon transcription and translation. The proaleurain signal peptide will direct the urokinase to the plant cell vacuole, but is itself cleaved off during transit through the plant endomembrane system to generate the mature protein.

In another non-limiting embodiment, a signal peptide may be engineered into the expression construct to direct the urokinase to be secreted from the plant cell. For example, and not by way of limitation, the signal peptide of tobacco PR-i, which is a secreted pathogenesis-related protein (Cornelissen *et al*., 1986, EMBO J. 5:37-40), can be engineered into the expression construct to direct the secretion of the urokinase from the plant cell. Alternatively, the potato patatin signal peptide (Iturriaga *et al.*, *Plant C*e*ll* 1:381-390 (1989) may be used to direct secretion of UK from the cell. Other methods for achieving secretion of recombinant proteins from plant cells are known in the art.

In an additional non-limiting embodiment, the signal peptide may be engineered into the expression construct to direct the urokinase to be retained within the ER. Such ER-retained urokinases may exhibit altered, and perhaps preferable, glycosylation patterns as a result of failure of the peptide to progress through the Golgi apparatus, thus resulting in a lack of subsequent glycosyl processing. For example, and not by way of limitation, a nucleotide sequence can be engineered into the expression construct to result in fusion of the amino acid sequence KDEL, i.e., Lys-Asp-Glu-Leu, to the carboxyl-terminus of the urokinase. The KDEL sequence results in retention of the urokinase in the ER (Pfeffer and Rothman, *Ann. Rev. Biochem.* 56:829-852 (1987)).

The expression construct may be further modified according to methods known to those skilled in the art to add coding sequences that facilitate purification of the urokinase. In one non-limiting embodiment, a nucleotide sequence coding for the target epitope of a monoclonal antibody may be engineered into the expression construct in operative association with the regulatory elements and situated so that the expressed epitope is fused to the urokinase. For example, and not by way of limitation, a nucleotide sequence coding for the FLAG™ epitope tag (International Biotechnologies, Inc. IBI), which is a hydrophilic marker peptide, can be inserted by standard techniques into the expression construct at a point corresponding to the carboxyl-terminus of the urokinase. The expressed FLAG™ epitope-urokinase fusion product may then be detected and affinity-purified using anti-FLAG™ antibodies. In an alternative embodiment, a nucleotide encoding a string of histidine residues (for example, six histidines) can be added at the N- or C-terminus of the urokinase. The urokinase (His)₆ fusion protein then may be purified using a nickel chelate column, which binds to the poly-His motif. *See* Tang *et al.*, *Protein Expression and Purification* 11:279 (1997). In one embodiment, the polynucleotide encoding the poly-His motif is appended to the urokinase-encoding sequence via a linker sequence that encodes a selective protease cleavage site. Suitable protease cleavage sites include enterokinase cleavage sites, and tobacco etch virus protease cleavage sites. Other selective protease cleavage sites are well known in the art.

In another non-limiting embodiment, a nucleotide sequence can be engineered into the expression construct to provide for a cleavable linker sequence between the urokinase peptide sequence and any targeting signal, reporter peptide, selectable marker, or detectable marker, as described *supra,* that has not otherwise been cleaved from the urokinase peptide sequence during peptide processing and trafficking through the plant endomembrane system. Such a linker sequence can be selected so that it can be cleaved either chemically or enzymatically during purification of the urokinase (Light *et al., Anal. Biochem.* 106:199-206 (1980)).

### Plant transformation vectors

The transformation vectors of the invention may be developed from any plant transformation vector known in the art including, but are not limited to, the well-known family of Ti plasmids from *Agrobacterium* and derivatives thereof, including both integrative and binary vectors, and including but not limited to pBIB-KAN, pGA471, pEND4K, pGV38SO, and pMONSOS. Also included are DNA and RNA plant viruses, including but not limited to CaMV, geminiviruses, tobacco mosaic virus, and derivatives engineered therefrom, any of which can effectively serve as vectors to transfer a urokinase coding sequence, or functional equivalent thereof, with associated regulatory elements, into plant cells and/or autonomously maintain the transferred sequence. In addition, transposable elements may be utilized in conjunction with any vector to transfer the coding sequence and regulatory sequence into a plant cell.

To aid in the selection of transformants and transfectants, the transformation vectors may preferably be modified to comprise a coding sequence for a reporter gene product or selectable marker. Such a coding sequence for a reporter or selectable marker should preferably be in operative association with the regulatory element coding sequence described supra.

Reporter genes which may be useful in the invention include but are not limited to the '3-glucuronidase (GUS) gene (Jefferson *et al*., *Proc. Natl. Acad. Sci. USA*, 83:8447 (1986)), and the luciferase gene (Ow et al. , Science 234:856 (1986)). Coding sequences that encode selectable markers which may be useful in the invention include but are not limited to those sequences that encode gene products conferring resistance to antibiotics, anti-metabolites or herbicides, including but not limited to kanamycin, hygromycin, streptomycin, phosphinothricin, gentamicin, methotrexate, glyphosate and sulfonylurea herbicides, and include but are not limited to coding sequences that encode enzymes such as neomycin phosphotransferase II (NPTII), chloramphenicol acetyltransferase (CAT), and hygromycin phosphotransferase I (HPT, HYG).

### Transformation/transfection of plants

A variety of plant expression systems may be utilized to express the urokinase coding sequence or its functional equivalent. Particular plant species may be selected from any dicotyledonous, monocotyledonous species, gymnospermous, lower vascular or non-vascular plant, including any cereal crop or other agriculturally important crop. Such plants include, but are not limited to, alfalfa, *Arabidopsis,* asparagus, barley, cabbage, carrot, celery, corn, cotton, cucumber, flax, lettuce, oil seed rape, pear, peas, petunia, poplar, potato, rice, beet, sunflower, tobacco, tomato, wheat and white clover.

Methods by which plants may be transformed or transfected are well-known to those skilled in the art. See, for example, Plant Biotechnology, 1989, Kung & Arntzen, eds., Butterworth Publishers, ch. 1, 2. Examples of transformation methods which may be effectively used in the invention include but are not limited to *Agrobacterium-mediated* transformation of leaf discs or other plant tissues, microinjection of DNA directly into plant cells, electroporation of DNA into plant cell protoplasts, liposome or spheroplast fusion, microprojectile bombardment, and the transfection of plant cells or tissues with appropriately engineered plant viruses.

Plant tissue culture procedures necessary to practice the invention are well-known to those skilled in the art. See, for example, Dixon, 1985, Plant Cell Culture: A Practical Approach, IRL Press. Those tissue culture procedures that may be used effectively to practice the invention include the production and culture of plant protoplasts and cell suspensions, sterile culture propagation of leaf discs or other plant tissues on media containing engineered strains of transforming agents such as, for example, *Agrobacterium* or plant virus strains and the regeneration of whole transformed plants from protoplasts, cell suspensions and callus tissues.

The invention may be practiced by transforming or transfecting a plant or plant cell with a transformation vector containing an expression construct comprising a coding sequence for the urokinase and selecting for transformants or transfectants that express the urokinase. Transformed or transfected plant cells and tissues may be selected by techniques well-known to those of skill in the art, including but not limited to detecting reporter gene products or selecting based on the presence of one of the selectable markers described supra. The transformed or transfected plant cells or tissues are then grown and whole plants regenerated therefrom. Integration and maintenance of the urokinase coding sequence in the plant genome can be confirmed by standard techniques, e.g., by Southern hybridization analysis, PCR analysis, including reverse transcriptase-PCR (RT-PCR) or immunological assays for the expected protein products. Once such a plant transformant or transfectant is identified, a non-limiting embodiment of the invention involves the clonal expansion and use of that transformant or transfectant in the production of urokinase.

As one non-limiting example of a transformation procedure, Agrobacterium-mediated transformation of plant leaf disks can follow procedures that are well known to those skilled in the art. Briefly, leaf disks can be excised from xenically grown plant seedlings, incubated in a bacterial suspension, for example, 10⁹ cfu/ml, of *A. tumefaciens* containing an engineered plasmid comprising a selectable marker such as, for example, kanamycin resistance, and transferred to selective shoot regeneration medium containing, for example, kanamycin, that will block growth of bacteria and untransformed plant cells and induce shoot initiation and leaf formation from transformed cells. Shoots are regenerated and then transferred to selective media to trigger root initiation. Stringent antibiotic selection at the rooting step is useful to permit only stably transformed shoots to generate roots. Small transgenic plantlets may then be transferred to sterile peat, vermiculite, or soil and gradually adapted for growth in the greenhouse or in the field.

### Identification and purification of the urokinase gene product

Transcription of the urokinase coding sequence and production of the urokinase in transformed or transfected plants, plant tissues, or plant cells can be confirmed and characterized by a variety of methods known to those of skill in the art. Transcription of the urokinase coding sequence can be analyzed by standard techniques, including but not limited to detecting the presence of urokinase messenger ribonucleic acid (mRNA) transcripts in transformed or transfected plants or plant cells using Northern hybridization analysis or RT-PCR amplification.

Detection of the urokinase itself can be carried out using any of a variety of standard techniques, including, but not limited to, detecting urokinase activity in plant extracts, *e.g.*, by detecting hydrolysis either of plasminogen or a plasminogen analogue. Additionally, the urokinase can be detected immunologically using monoclonal or polyclonal antibodies, or immuno-reactive fragments or derivatives thereof, raised against the protein, *e.g.,* by Western blot analysis, and limited amino acid sequence determination of the protein.

Indirect identification of enzyme production in a plant can be performed using any detectable marker or reporter linked to the urokinase. For example, but not by way of limitation, FLAG™ epitope, which can be linked to the urokinase, as described supra, is detectable in plant tissues and extracts using anti-FLAG M2 monoclonal antibodies™ (IBI) in conjunction with the Western Exposure^{TM} chemi-luminescent detection system (Clontech) .

Urokinase production in a transformed or transfected plant can be confirmed and further characterized by histochemical localization, the methods of which are well-known to those skilled in the art. See, for example, Techniques in Immunocytochemistry, Vol I, 1982, Bullock and Petrusz, eds., Academic Press, Inc. For example, but not by way of limitation, either fresh, frozen, or fixed and embedded tissue can be sectioned, and the sections probed with either polyclonal or monoclonal primary antibodies raised against the urokinase or, for example, antiFLAG™ monoclonal antibodies. The primary antibodies can then be detected by standard techniques, *e.g.*, using the biotinylated protein A-alkaline phosphatase-conjugated streptavidin technique, or a secondary antibody bearing a detectable label that binds to the primary antibody.

The expression products can be further purified and characterized as described in the subsections below.

### Production and purification of the urokinase gene product

One non-limiting method to produce and purify the urokinase is described here, wherein the urokinase coding sequence is operably associated with an inducible promoter in the expression construct. Leaf or other tissue or cells from a transgenic plant or cell culture transformed or transfected with this expression construct can be processed to induce expression of the urokinase coding sequence. This induction process may include inducing the activation of UK genes by one or more methods applied separately or in combination, including but not limited to physical wounding or other mechanical gene activation (MGA), and application of chemical or pathogenic elicitors or plant hormones. Methods of mechanical gene activation are described in U.S. Patent No. 5,670,349.

UK gene activation levels may also be enhanced in plaint cells or tissues by factors such as the availability of nutrients, gases such as O₂ and CO₂, and light or heat. After induction, the tissue may be placed in incubation media (50.mM sodium phosphate, pH 8, 150 mM NaCl), or maintained in the absence of media at room temperature. The tissue or induction media then can be stored, *e.g.*, at -20°C. If the UK protein is targeted for localization within the plant cell, the plant cell wall must be penetrated to extract the protein. Accordingly, the plant tissue can be ground to a fine powder, e.g., by using a cissue grinder and dry ice, or homogenized with a ground glass tissue homogenizer. The UK can be resuspended in incubation media. The homogenate can then be clarified by, for example, centrifugation at 10,300 x g for 30 mm to produce a cell-free homogenate.

The urokinase must be further purified if it is to be useful as a therapeutic or research reagent. The urokinase can be purified from plant extracts according to methods well-known to those of skill in the art (Wallen *et al., Biochemica et Biophysica Acta* 719:318 (1982); Husain *et al.*, *Arc.* Biochem *Biophys* 220:31 (1983); Wu *et al., Biochem.* 16:1908 (1977))). Once the presence of the enzyme is confirmed it can be isolated from plant extracts by standard biochemical techniques including, but not limited to, differential ammonium sulfate (AS) precipitation, gel filtration chromatography or affinity chromatography, *e.g.*, utilizing hydrophobic, immunological or lectin binding. At each step of the purification process the yield, purity and activity of the enzyme can be determined by one or more biochemical assays, including but not limited to: (1) detecting hydrolysis of the enzyme's substrate or a substrate analogue; (2) immunological analysis by use of an enzyme-linked immunosorbent assay (ELISA); (3) sodium dodecyl sulfate polyacrylamide gel electrophoresis (SDS-PAGE) analysis; and (4) Western analysis. The enzyme may be alternatively or additionally purified by affinity chromatography wherein the enzyme binds to its inhibitor which is linked, for example, to an inert substrate.

Once solubilized, all urokinase-containing fractions can be maintained, for example, by storage at 4°C, and stabilized if necessary, *e.g.*, glycerol or ethylene glycol.

### Proteolytic processing of the signal peptide

In order to address whether the plant expression system efficiently recognizes and correctly cleaves the human signal peptide from the urokinase, the plant-produced enzyme can be purified and analyzed by N-terminal sequencing. Accordingly, the enzyme can, for example, be treated with Endo-F/N-glucanase (Boehringer Mannheim) to remove N-linked glycans, and the resulting peptide can be repurified by methods described supra. The purity of the enzyme can be determined based, for example, on silver-stained SDS-PAGE. The band containing the enzyme can be excised from the gel, the peptide eluted therefrom, and then analyzed by commercial N-terminal amino acid sequencing to determine whether the correct cleavage of the signal peptide has occurred. Incomplete cleavage can be detected, for example, as a double band on SDS-PAGE, or as mixed N-terminal sequences.

### N-linked glycosylation in plants versus animals

The oligosaccharides of native human and animal urokinases are typical antennary structures containing N-acetylglucosamine, mannose, and sialic acid. The glycoconjugate associated with the urokinase of the invention may be determined, for example, by lectin binding studies (Reddy *et al., Biochem. Med.* 33:200-210 (1985), Cummings, *Meth. Enzymol.* 230:66-86 (1994)).

Plant glycans do not contain sialic acid, which is a prevalent terminal sugar in mammalian glycans. In addition, the complex glycans of plants are generally smaller than their animal counterparts and contain a ß 1-2 xylose residue attached to the β-linked mannose residues of the core (Gomez and Chrispeels, 1994, Proc. *Natl. Acad. Sci. USA* 91:1829-1833).

Determination of the glycan composition and structure of the urokinase of the invention is of particular interest because: (a) the glycan composition will indicate the status of the protein's movement through the Golgi; and (b) the presence of a complex glycan may indicate whether an antigenic response will be triggered in humans.

Several molecular, genetic and chemical approaches can be used to raise the proportion of the high-mannose form of glycans on urokinases, thereby making them more similar in structure to the native human protein (Berg-Fussman *et al.*, *J. Biol. Chem.* 268:14861-14866 (1993)). For example, but not by way of limitation, the mannose analog, 1-deoxymannonojirimycin (dMM), inhibits mannosidase I, the first Golgi-specific enzyme involved in glycan processing. Plant tissues treated with dMM produce glycoproteins which lack fucose and xylose and maintain a glycan profile consistent with inhibition at the mannosidase *I* step (Vitale *et al., Pl. Phys.* 89:1079-1084 (1989)). Treatment of urokinase-expressing plant tissues with dMM may be useful to produce urokinases with a relatively homogeneous high-mannose glycan profile. Such urokinases should be highly effective for use in thrombolytic treatment of humans and animals.

### Clonal propagation and breeding of transgenic plants

Once a transformed or transfected plant is selected that produces a useful amount of the recombinant urokinase of the invention, one embodiment of the invention contemplates the production of clones of this plant either by well-known asexual reproductive methods or by standard plant tissue culture methods. For example, tissues from a plant of interest can be induced to form genetically identical plants from asexual cuttings. Alternatively, callus tissue and/or cell suspensions can be produced from such a plant and subcultured. An increased number of plants subsequently can be regenerated therefrom by transfer to an appropriate regenerative culture medium.

Alternatively, the recombinant urokinase producing plant may be crossed as a parental line, either male or female, with another plant of the same species or variety, which other plant may or may not also be transgenic for the UK coding sequence, to produce an F1 generation. Members of the F1 and subsequent generations can be tested, as described supra, for the stable inheritance and maintenance of the urokinase coding sequence, as well as for urokinase production. A breeding program is thus contemplated whereby the urokinase coding sequence may be transferred into other plant strains or varieties having advantageous agronomic characteristics, for example, by a program of controlled backcrossing. The invention thus encompasses parental lines comprising the urokinase coding sequence, as well as all plants in subsequent generations descending from a cross in which at least one of the parents comprised the urokinase coding sequence. The invention further encompasses all seeds comprising the urokinase coding sequence and from which such plants can be grown, and tissue cultures, including callus tissues, cell suspensions and protoplasts, comprising the urokinase coding sequence, whether or not they can be regenerated back to plants.

### Transient expression in tobacco cell cultures

Another feature of the plant expression system is the transient expression of UK in plant cells (An, Plant *Plysiol.* 79:568 (1985). As one example, this system involves *Agrobacterium-*mediated introduction of DNA but analysis of expression is not dependent upon integration. In this procedure, acetysyringone-activated Agrobacterium carrying the engineered vector carrying UK gene is co-cultivated in liquid medium with N. tabacum cells. Six hours later, expression of the UK gene can be induced by the addition of cellulysin, an enzyme which releases oligosaccharide components from the plant cell wall that trigger host defense responses including MEGA induction. Using MeGA:reporter gene constructs, it has been demonstrated that between 30 and 50% of the tobacco cells show significant transgene expression 24 hours after induction. For transient expression of the transgene, the product may be isolated from the cells or from the culture media if secreted.

### Methods for therapeutic use of urokinase

The recombinant urokinases of the invention are useful for thrombolytic treatment by providing a therapeutic amount of urokinase, or a derivative or modification thereof, to a patient suffering from an arterial or venous blockage caused by the presence of a fibrin clot.

By "therapeutic amount" is meant an amount of biologically active urokinase that will cause significant alleviation of clinical symptoms caused by the presence of the fibrin clot.

A therapeutic amount causes "significant alleviation of clinical symptoms" caused by the presence of a clot if it serves to reduce one or more of the pathological effects or symptoms of the disease or to reduce the rate of progression of one or more of such pathological effects or symptoms.

Methods and dosage regimens for administering urokinase in a therapeutic setting are well known in the art. For example, urokinase purified from cultures of human kidney cells is commercially available and has been approved by the Food and Drug Administration (FDA) for treatment of pulmonary embolism and coronary artery thrombosis. The dosages and therapeutic regimens used for administering the urokinase of the invention are expected to be similar to those approved by the FDA for the commercially available urokinase. Any optimization of therapeutic regimen required for use of the urokinase of the invention will require.experimentation that is routine for the skilled physician.

Alternatively, effective dosage and treatment protocol may be determined by conventional means, starting with a low dose in laboratory animals and then increasing the dosage while monitoring the effects, and systematically varying the dosage regimen as well. The amount of recombinant urokinase to be administered to a patient suffering from an arterial or venous blockage will vary. Numerous factors may be taken into consideration by a clinician when determining an optimal dose for a given subject. These factors include the size of the patient, the age of the patient, the general condition of the patient, the particular disease being treated, the severity of the blockage, the presence of other drugs in the patient, and the like. Trial dosages would be chosen after consideration of the results of animal studies, and available clinical literature with respect to past results of thrombolytic therapy using urokinase.

For example, therapeutic amounts of recombinant UK and modified UK produced according to the invention may in each instance encompass dosages of about 2,000 I.U./kg/hr, but higher or lower doses may be used depending upon the severity of the blockage.

The amount of recombinant urokinase of the invention administered to the patient may be decreased or increased according to the enzymatic activity of the particular urokinase. For example, administration of a recombinant urokinase of the invention which has been modified to have increased enzymatic activity relative to the native human or animal enzyme will require administration of a lesser amount to the patient than a native human or animal urokinase having lower enzymatic activity.

In addition, the amount of recombinant urokinase administered to the patient may be modified over time depending on a change in the condition of the patient as thrombolysis progresses, the determination of which is within the skill of the attending clinician.

The invention also provides pharmaceutical formulations for use of the recombinant urokinase in treating arterial or venous blockages. The formulations comprise a recombinant urokinase of the invention and a pharmaceutically acceptable carrier. A variety of aqueous carriers may be used, *e.g.,* water, buffered water, 0.4% saline, 0.3% glycine, and the like. The pharmaceutical formulations may also comprise additional components that serve to extend the shelf-life of pharmaceutical formulations, including preservatives, protein stabilizers, and the like. The formulations are preferably sterile and free of particulate matter (for injectable forms) . These compositions may be sterilized by conventional, well-known sterilization techniques.

The compositions may contain pharmaceutically acceptable auxiliary substances as required to approximate physiological conditions, such as pH adjusting and buffering agents, toxicity adjusting agents and the like, e.g., sodium acetate, sodium chloride, potassium chloride, calcium chloride, sodium lactate, etc.

The formulations may be adapted for various forms of administration, including intramuscularly, subcutaneously, intravenously and the like. The subject formulations may also be formulated so as to provide for the sustained release of a urokinase. Actual methods for preparing parenterally administrable compositions and adjustments necessary for administration to subjects will be known or apparent to those skilled in the art and are described in more detail in, for example, Remington's Pharmaceutical Science, 17th Ed., Mack Publishing Company, Easton, Pa. (1985), which is incorporated herein by reference.

The present invention, thus generally described, will be understood more readily by reference to the following examples, which are provided by way of illustration and are not intended to be limiting of the present invention.

### EXAMPLE 1: Production and isolation of recombinant modified urokinase from transgenic tobacco plants

The subsections below describe the production of a biologically active urokinase in tobacco. An *Escherichia* *coli* strain containing a full-length cDNA for preprourokinase was obtained from J.D. Sato (Adirondack Biomedical Research Institute, Lake Placid, NY). The cDNA was obtained by standard RT-PCR procedures using template RNA extracted from the A431 human epidermoid carcinoma cell line. A431 cells are available from, *inter alia,* ATCC. Briefly, total mRNA was isolated from A431 cells and used as a template for cDNA synthesis according to the manufacturer's recommendation (Perkin-Elmer, RT-PCR Cloning Kit). 5' and 3' oligonucleotide primers specific to human preprourokinase were used to generate the urokinase cDNA. The primers contained added XbaI sites to aid cloning of the PCR products. The resultant cDNA was cloned into the pBluescript II phagemid (Stratagene, La Jolla, CA) and transfected into *E. coli* DH5-alpha cells (Life Technologies). The sequence of the urokinase cDNA was determined by the dye-labeled dideoxynucleotide termination method on a model 370A automated DNA sequencer (Applied Biosystems). Point mutations were corrected by PCR using oligonucleotide primers and polymerase mixture of *Taq* and Vent™ DNA polymerases (New England BioLabs) according to the manufacturer's recommendations.

Three modifications of UK cDNAs were constructed and expressed in transgenic plants. First, a native human preUK cDNA was constructed and placed under the transcriptional control of the MeGA promoter to generate pCT92. Second, the human signal peptide was replaced with a potato patatin signal peptide and fused with UK behind the MeGA promoter to generate pCT111. Third, UK lacking a signal peptide was placed under the control of the MeGA promoter to generate pCT97.

The MeGA promoter, comprising a 456 bp fragment (FIG. 3) as modified from the tomato HMG2 promoter (Weissenborn *et al.*, *Phys. Plantarum* 93:393-400 (1995)), was used to drive the expression of the urokinase gene. The MeGA promoter is inducible and has a low basal expression in unstressed plant tissues, but is highly induced in both immature and mature tissues by the process of mechanical gene activation (MGA), or by a variety of chemicals that induce plant defense responses. MGA includes but is not limited to the mechanical shredding of leaf tissue, for example, into 2 mm strips, followed by storage at room temperature on Whatman 3MM chromatography paper moistened with sterile water in a sealed plastic bag. The expression of a MeGA:GUS construct has been monitored in transgenic tobacco plants from seedling stage to flowering. No loss of inducible activity was observed as plants reached maturity.

### Cloning of the urokinase cDNA into MeGA plant expression vector:PCT92.

### Promoter:urokinase expression construct

A 5' 418 bp fragment was generated by a polymerase chain reaction using oligonucleotide primers UK1 and UK2 (TABLE 2) with preUK cDNA as template. The PCR product was isolated by agarose gel elution and digested with XbaI, treated with mungbean nuclease I (Stratagene. La Jolla, CA) to blunt end the site, followed by digestion with PstI to generate a 364 bp 5' blunt-ended/3' PstI fragment. This fragment was ligated to the PstI digested pCT73 plasmid containing the 3' region of the urokinase cDNA contained within a 0.96 kb PstI/SacI fragment. A MeGA promoter plasmid containing the start codon, AUG, was digested with PstI and treated with mung bean nuclease to blunt end the site, followed by digestion with SacI to generate a 498 bp 5' SacI /3' blunt ended fragment, that then was ligated to the preUK cDNA (Figure 4).

The resulting 1.8 kb SacI fragment containing the MeGA:preUK cDNA was ligated to pBluescript (KS)II that had been digested with SacI. Two independent pBluescript clones containing the MeGA:preUK plasmid were sequenced using T7 (Stratagene, SK (Stratagene) and UK2 primers, confirming that the sequence was correct. The 1.8 kB SacI fragment from these plasmids was ligated into the plant transformation vector, pBIB-Kan' (Becker, *Nucl*. *Acids. Res.,* 18:203 (1990) (Figure 4). plasmids having the desired orientation in pBIB-Kan were identified by restriction analysis with XbaI. The correct orientation of the 1.8 kb SacI fragment was expected to yield a 0.5 kb XbaI fragment, whereas the incorrect orientation was expected to produce a 1.3 kb XbaI fragment (Figure 4). The resulting pBIB-Kan: MeGA™:preUK plasmids, designated pCT92, were used to generate transgenic plants.

**TABLE 2.**

| **PCR Primers to Human Preprourokinase cDNA** | | |
|---|---|---|
| Primer | Restriction site linker | Sequence |
| UK1 | XbaI | 5' GC[TCTAGa]gagccctg2ctggcgcg 3' |
| UK2 | None | 5' gcccacctgcacatagcaccag 3' {25 bp 3' of internal *PstI* site, Figure 4) |
| UK3 | *xba*I | 5' GC[TCTAGa]gcaattgaacttcatcaagt 3' |

### Cloning of prourokinase lacking the human signal peptide into the MeGA:patatin signal peptide plant expression vector

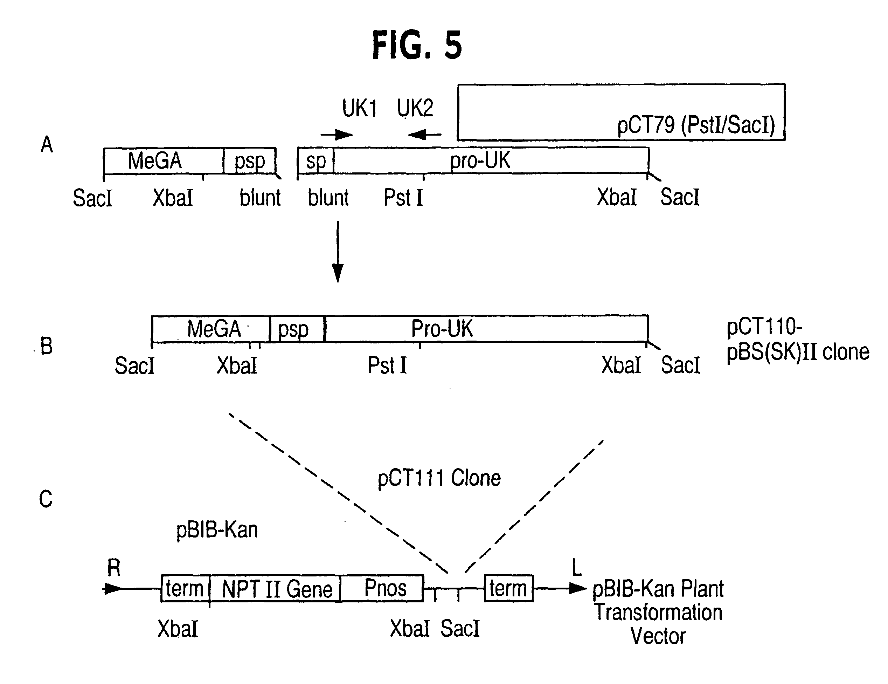

To attempt to maximize the potential amounts of UK expressed in tobacco, the human signal peptide was replaced with a plant signal peptide from patatin protein (PSP). The patatin signal peptide (MSPPKSFLILFFMILAPPSSTCA) has been shown to be efficiently processed in tobacco plants (Bevan *et al*., *Nuc*. *Acids Res.* 14:4625-4638 (1986); Iturriaga *et al*., *The Plant Cell* 1:381 (1989)). As shown in Figure 5, the 5' region of prourokinase (minus the human signal peptide) was generated by PCR of template human preUK cDNA using oligo-primers UK2 and UK3, and PCR. This 361 bp fragment was digested with XbaI and treated with mung bean nuclease to blunt end the site, followed by digestion with PstI to generate a 307 bp 5' blunt-end/3' PstI fragment. This was ligated to the PstI-digested pCT79 clone generated as described above. The clone containing the MeGA:PSP fragment (Figure 4 was digested with *Kpn*I and treated with mung bean nuclease to blunt end the site, generating a 567 bp 5' SacI/3' blunt-ended fragment. This fragment then was ligated to the UK cDNA fragment to generate a 1.9 kb SacI fragment. This SacI fragment then was cloned into the pBS(SK)II vector to produce a vector, designated pCT110, that was subjected to sequence analysis using the T7, SK and UK2 primers. The cloning junctions were sequenced and found to contain no alteration.

The 1.9 SacI fragments from two independent plasmids were isolated and ligated to the plant transformation vector, pBIB-Kan. The plasmids with the correct orientation in pBiB-Kan were determined by restriction analysis as described above. The pBIB-Kan:MeGA:patatin signal peptide/UK clones, designated pCT111, were used to generate transgenic plants.

### Cloning of urokinase lacking a signal peptide into the MeGA:plant expression vector

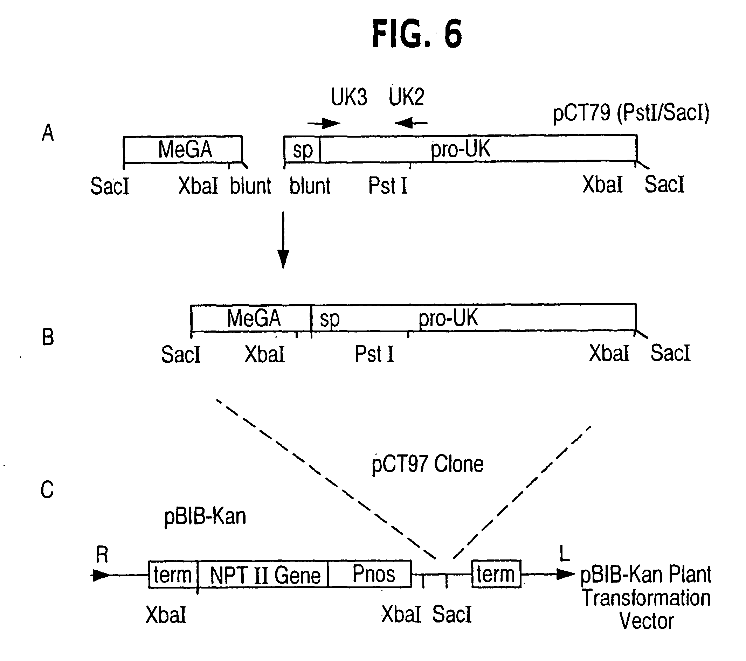

As described in Figure 5, the 5' region of urokinase (minus the human signal peptide) was generated and ligated to the PstI-digested pCT79 plasmid. A MeGA promoter clone containing the start codon, AUG, was digested with PstI and treated with mung bean nuclease to blunt end the site, followed by digestion with SacI to generate a 498 bp 5' SacI/3' blunt ended fragment. This fragment then was ligated to the UK cDNA (Figure 6) to generate a 1.7 kb SacI fragment. The *Sac*I fragment was cloned into pBS(SK)II for sequence analysis, using the T7, SK and UK2 primers. The cloning junctions were sequenced and found to contain no alteration. The 1.7 kB SacI fragments from two independent plasmids were isolated and ligated to the plant transformation vector, pBIB-Kan (Figure 6). The resulting pBIB-Kan: meGA:uK plasmids, designated pCT97, were used to generate transgenic plants.

### Stable integration of the UK into tobacco via Agrobacterium-mediated transformation

Leaf disks were excised from aseptically grown tobacco seedlings (*Nicotiana tabacum* cvs. Xanthi a non-commercial variety) and briefly incubated in a bacterial suspension (10⁹ cfu/ml) of *A*. *tumefaciens* containing the engineered plasmid. The discs then were co-cultivated on plates containing a nurse-culture of cultured tobacco cells for 48 hr, and then transferred to selective "shooting" medium (MS media; Murashige & Skoog, 1962, *Physiol Plant.* 15:473-497) , containing 100 mg/L kanamycin and 9.12 µM zeatin) which blocks growth of bacteria and untransformed plant cells and induces shoot initiation and leaf formation from transformed cells (Horsch *et al*. , Science 223:496-498 (1984)). Prolific shoots were developed by three weeks and transferred to selective media which triggered root initiation (MS media, 100 mg/L kanamycin, 10 µM indole-3-acetic acid). Stringent antibiotic selection at the rooting step permits only stably transformed shoots to generate roots. Small transgenic plantlets were transferred to sterile peat cups and subsequently to potting mix for growth in the greenhouse. Transgenic leaf material was available for initial testing by about 2 to 3 months after initial cocultivation.

| **TABLE 3: Generation of transgenic plants expressing UK** | | |
|---|---|---|
| Construct | Gene | No. of Transgenic Plants |
| CT92 | human preUK | 55 |
| CT111 | plant signal peptide:UK | 87 |
| CT97 | UK | 45 |

### Biological activity in tobacco extracts

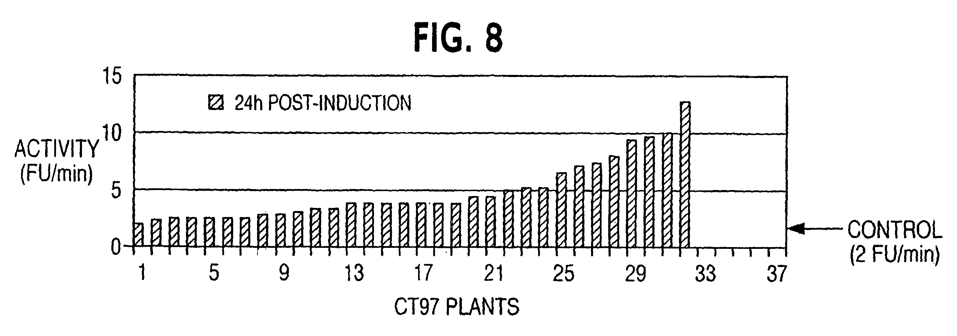

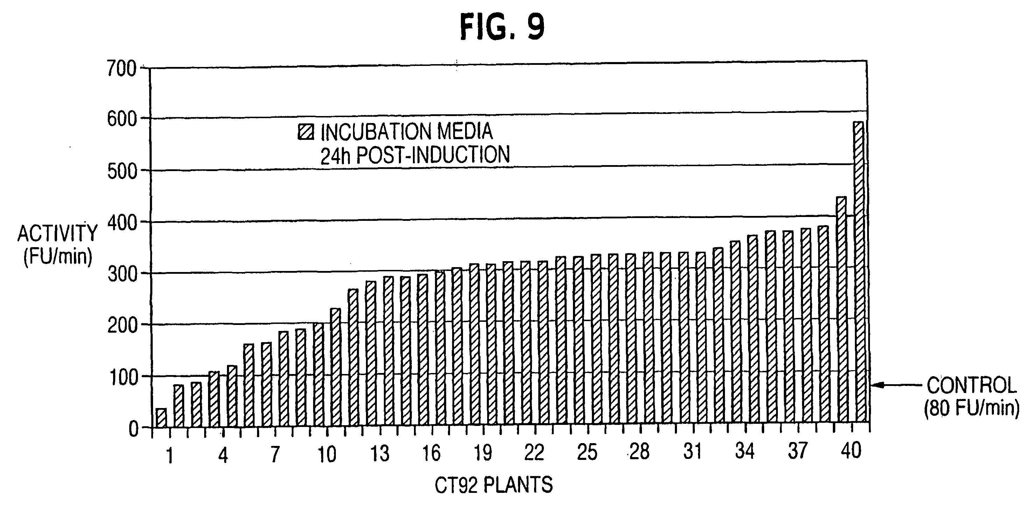

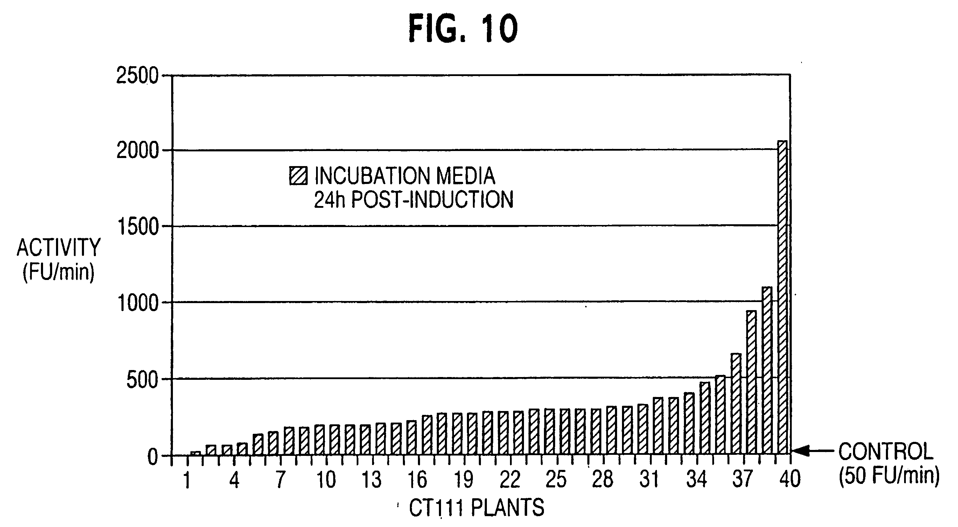

Transgenic plant tissue was tested for UK activity using a fluorogenic peptide assay (Kato *et al., J. Biochem* 88:183 (1980)), a modified zymography plasminogen activator assay (Vassalli et al., J. *Exp. Med.* 159:1653 (1984) and a SDS-polyacrylamide "in-gel" activity assay (Roche *et al*., *Biochem. Biophys. Acta* 745:82 (1983) for plasminogen activators. These assays are widely used to determine the enzymatic activity of plasminogen activators as well as plasmin. The fluorogenic peptide assay uses the substrate N-t-Boc-Ile-Glu-Gly-Arg-7-amido-4-methylcoumarin (NtB)(Sigma Co., St. Louis, MO, cat. no. B9860). At an excitation wavelength of 380 nm, an increase in emission at 470 nm is observed when NtB is cleaved, indicating the presence of enzymatic activity. NtB also serves as a substrate for endogenous plant nonspecific proteases with very low specificity for NtB which have been detected in leaves of both the control non-transformed plants and transgenic plants. Transgenic plants containing recombinant UK gene were distinguished from plants that were not transformed with UK gene by the higher level of UK activity found after induction (Figure 8, 9, 10). Using the modified zymography assay, no endogenous UK like activity was found in control plants, and only the transgenic UK plants had plasminogen activator activity. Briefly, eight mls of 1% agarose containing 0.1 M Tris pH 8, 0.6% (w/v) non-fat dry milk, 0.01% (w/v) sodium azide and 0.03 IU/ml human plasminogen (Sigma) was poured into a 90 cm petri plate to solidify. Wells were punched and 10-15 µl of sample or control urine-derived HMW UK at 0.25, 0.5, 1, 10, 50 IU/ml was placed into the wells. The plates were incubated at 37°C for 10-16h. A zone of clearing was only present in the CT92 and CT111 plant samples induced for 24 h and the control urine derived HMW UK. This indicated that the samples from transgenic UK plants had plasminogen activator activity. To determine the molecular weight forms which had activity, a urokinase "in-gel" activity assay was performed. A zone of clearing was detected at 52 kD and 22 kD in samples from transgenic UK plants. This indicated that both the transgenic SC-UK and the lower molecular weight form had plasminogen activation activity.

The urokinase "in-gel" activity assay was performed with a polyacrylamide resolving gel containing 10% acrylamide (acrylamide:bisacrylamide ration 29.2%:0.8%), 0.375 M Tris pH 8.6, 0.1% SDS, 0.2% casein (Biorad), 150 µg/ml porcine plasminogen (Sigma) and a 4.5% stacking gel (pH 6.8). Protein samples were equilibrated in sample buffer (0.0625 M Tris, 10% glycine, 2.5% SDS pH 6.8) and loaded and run at room temperature at constant 20 mA for 4 hrs. The gels were washed in an excess of 2.5% Triton X100 for 1 hr, rinsed in 0.050 M Tris, 0.1M NaCl pH 7.6 and incubated at 37°C for 6 h in 150 ml buffer. After incubation the gels were trained for 10 min (0.25% Coomassie blue R-250, 50% methanol, 7% acetic acid) and destained (30% methanol, 10% acetic acid) for 16 hr. This procedure allowed direct identification of the approximate molecular size of active UK.

The fluorogenic peptide assay used a 2 ml reaction volume with assay buffer (50 mM Tris pH 8.8., 150 mM NaCl; , 0.1 %PEG, 0.05% Triton X 100, 50 µM NtB), without plasmin or with plasmin (10⁴ U/ml porcine plasmin) and 0 to 0.5, ml of sample. The samples were placed in a DyNA Quant™ 200 Fluorometer (Hoefer Pharmacia Biotech Inc.) and readings were taken from 0 to 240 min. at room temperature. (Kato *et al*., supra).

### Transgenic UK is secreted

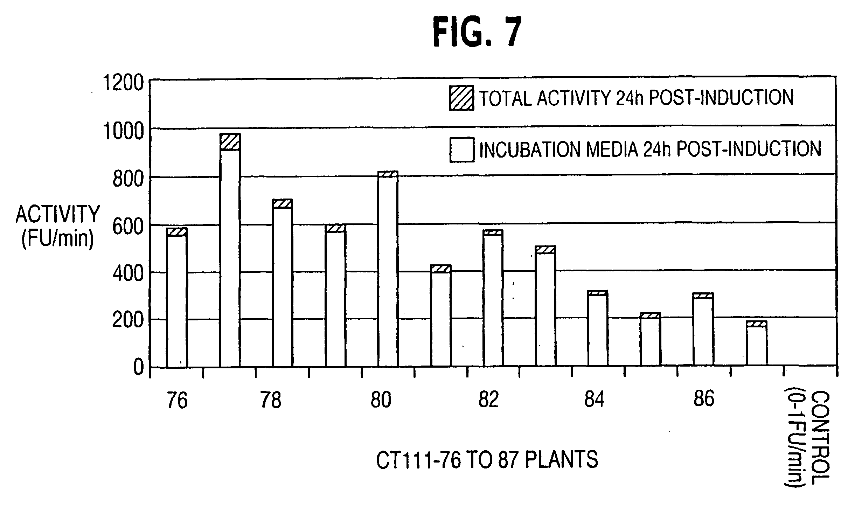

As discussed above, human UK is normally secreted from various sources including the kidney. In addition, both insect and mammalian cells secrete recombinant UK into the media. To determine if tobacco also secretes recombinant UK into the media, leaf tissue from transgenic UK plants (CT111-76 to 87) were induced for 24 hours in a plastic petri dish with filter sterilized incubation media (PBS: 50 mM sodium phosphate pH 8, 150 mM NaCl). At the same time leaf tissue from two control plants (pBiB-kan vector only) were treated in a similar fashion. The incubation media were collected and assayed. The induced leaf tissue was homogenized in extraction buffer and the supernatant collected. As shown in Figure 7, when transgenic plants expressing the patatin signal peptide/UK cDNA (CT111) were analyzed, the vast majority of the UK activity was found in the incubation media and not in the cell extract. When the plasminogen activator zymography assay was used to analyze transgenic UK, the control 0 h & 24 h post-induction and transgenic 0 h post-induction samples showed no UK activity - only the transgenic samples induced for 24 h showed UK activity. These results indicate that the transgenic UK plants express active human UK enzyme.

### Increased level of UK expression in transgenic plants with patatin signal/UK construct

Transgenic plants expressing three different gene constructs under the control of the MEGA promoter were analyzed. As shown in TABLE 3, the main difference between the three gene constructs is the nature of the signal peptide: CT92 has the human signal peptide, CT111 has the plant signal peptide, CT97 contained no signal peptide. From two grams of tissue (Figure 8, 9, 10), transgenic CT111 produced lines with the highest UK activity (100-2000 FU/min), followed by CT92 (100-600 FU/min). Transgenic CT97 had significantly less UK activity then the other transgenic lines (2-13 FU/min). Although patatin signal peptide has been used to express heterologous genes (Iturriaga *et al.*, *supra,* 1989, Sijmons *et al.*, *Bio*/*Technology* 8:217-221 (1990)), no direct comparison has been made between the native and patatin signal peptide on expression levels. The results described above indicate that, in the case of human UK, the patatin signal peptide significantly increases the level of UK activity in transgenic tobacco.

### Immunodetection of UK protein in transgenic plant extracts

Eight weeks after plants were potted in soil, 2 grams of leaf tissue from transgenic plants listed in TABLE 3 were harvested, induced and placed in 5 mls of filter sterilized PBS incubation media (50 mM sodium phosphate pH 8, 150 mM NaCl, PBS) at room temperature for 24 hours. The incubation medium was collected and centrifuged at 10,000 x G for 10 min to remove debris. The supernatant was spotted on nitrocellulose membrane for immuno-slot blot analysis. Fifty µl of supernatant was placed in an equal volume of incubation media and vacuum spotted onto a nitrocellulose membrane (MINIFOLD II slot blot system, Schleicher & Schuell, Keen, NH). One to five ng of urine-derived HMW UK (American Diagnostica Inc.) also was blotted and human UK was detected using rabbit polyclonal antibodies to human HMW UK (American Diagnostica Inc.; Kobayashi *et al.*, *J.Biol. Chem* 6:5147 (1991)) at 1:1000 dilution. Alkaline phosphatase-conjugated goat anti-rabbit polyclonal antibody (BioRad, Inc.) was used in the Western Exposure™ chemiluminescent detection system (Clontech, Inc.) to test for immunoreactive material. Of the 38 CT92 transgenic plants analyzed, 26 showed significant transgene expression. Of the 87 CT111 transgenic plants analyzed, 54 showed significant transgenic expression. Of the 27 CT97 transgenic plants analyzed, 6 showed significant transgenic expression.

Induction media and leaf extracts from induced leaves of untransformed plants and transformed plants CT92, CT97, and CT111 were tested for immuno-reactivity to monoclonal antibodies (American Diagnostics, Inc.) to human HMW UK. The protein samples were separated on non-reducing SDS-PAGE, transferred to membrane (Hoefer Western System, Hoefer Pharmacia Inc.), and probed with anti-HMW UK (dilution 1:100). Urine-derived HMW UK, 50 ng, was used as a control sample.

The anti-HMW UK did not cross react with endogenous plant proteins present in the incubation media but was specific to human UK. Anti-HMW UK exhibited a strong cross-reaction to bands with an apparent molecular weight of 64-70 kD in CT92, CT97 and CT111 plants. In CT92 and CT111 plants a 34 kD band which cross-reacted with the anti-HMW UK was observed. The urine-derived activated HMW UK had multiple bands showing a strong cross reaction at 54 kD, 36 kD, and 16 kD. Single-chain UK purified from various sources has been found to migrate at a range of sizes under non-reducing conditions (Husain et al., *Arch. Biochem. Biophys.* 220:31 (1983)). When transgenic UK was analyzed under reducing conditions containing 5-10% β-mercaptoethanol, a 52 kD protein cross-reacted with anti-HMW UK in all transgenic plant samples. A CT111 plant analyzed co-migrated with human single-chain UK at the molecular size of 52 kD indicating that tobacco produces single chain UK of the expected size. In addition, a second anti-UK cross-reacting species was detected in the molecular size range of 32-36 kD, a size similar to the "activated" or LMW UK form found in human urine. Control urine-derived two-chain HMW UK displayed bands at 31 kD and 20 kD bands that cross reacted with anti-HMW UK. These bands were derived from the two-chain human UK protein. No bands were observed in the transgenic plant samples that co-migrated with urine-derived HMW UK, indicating that the majority of the transgenic UK is not activated but remains in single chain form. This result is consistent with results obtained for UK purified from bacteria (Winkler and Blaber, Biochem. 25:4041 (1986)), yeast (Melnick et al., J. Biol. Chem. 265:801 (1990)), and mammalian cells (Kohno *et al.*, *Bio*/*Technology* 2:628 (1984)).

### DEPOSIT OF BIOLOGICAL MATERIALS

The plasmid pCT110, containing the MeGA promoter linked to the PSP signal peptide and a urokinase cDNA has been deposited with the American Type Culture Collection (ATCC) at 12301 Parklawn Drive, Rockville, MD. 20852, in compliance with the requirements of the Budapest Treaty On The International Recognition Of The Deposit Of Microorganisms For The Purpose Of Patent Procedure.

The present invention is not to be limited in scope by the biological material deposited since the deposited embodiments are intended as illustrations of the individual aspects of the invention, and any biological material, or constructs which are functionally equivalent are within the scope of this invention. Indeed various modifications of the invention in addition to those shown and described herein will become apparent to those skilled in the art from the foregoing description and accompanying drawings. Such modifications are intended to fall within the scope of the appended claims.

## Claims

1. A method for producing a biologically active urokinase in a transgenic plant, comprising:
(a) growing the transgenic plant which has a recombinant expression construct comprising a nucleotide sequence encoding the urokinase and a promoter that regulates expression of the nucleotide sequence so that the urokinase is expressed by the transgenic plant; and
(b) recovering the urokinase from an organ of the transgenic plant, wherein the organ is a leaf, stem, root, flower, fruit or seed.

2. The method according to claim 1, in which the promoter is an inducible promoter, and which method additionally comprises, between steps (a) and (b), the step of inducing the inducible promoter before or after the transgenic plant is harvested.

3. The method according to claim 2, in which the inducible promoter is induced by mechanical gene activation.

4. The method according to claim 3, in which the inducible promoter comprises the nucleotide sequence shown in Figure 3.

5. The method according to claim 1, in which the transgenic plant is a transgenic tobacco plant.

6. The method according to claim 1, in which the urokinase in a human urokinase.

7. The method according to claim 6, wherein said urokinase comprises amino acids 2-411 of the sequence shown in Figure 2c.

8. The method according to claim 1, wherein said expression construct encodes the amino acid sequence shown in Figure 2a.

9. The method according to claim 1, wherein said expression construct encodes the amino acid sequence shown in Figure 2b.

10. The method according to claim 1, wherein said expression construct comprises the nucleotide sequence shown in Figure 1a.

11. The method according to claim 10, wherein said expression product comprises the nucleotide sequence shown in Figure la under the control of the promoter shown in Figure 3.

12. The method according to claim 1, wherein said expression construct comprises the nucleotide sequence shown in Figure 1b.

13. The method according to claim 12, wherein said expression product comprises the nucleotide sequence shown in Figure 1b under the control of the promoter shown in Figure 3.

14. The method according to claim 1, wherein said expression construct comprises the nucleotide sequence shown in Figure 1c.

15. The method according to claim 14, wherein said expression product comprises the nucleotide sequence shown in Figure 1c under the control of the promoter shown in Figure 3.

16. A recombinant expression construct comprising a nucleotide sequence encoding a urokinase and a promoter that regulates the expression of the nucleotide sequence in a plant cell.

17. The recombinant expression construct of claim 16, in which the promoter is an inducible promoter.

18. The recombinant expression construct of claim 17, in which the inducible promoter is induced by mechanical gene activiation.

19. The recombinant expression construct of claim 18, in which the inducible promoter comprises the nucleotide sequence shown in Figure 3.

20. The recombinant expression construct of claim 16, in which the urokinase is a human urokinase.

21. A plant transformation vector comprising the recombinant expression construct of claim 20.

22. A plant cell, tissue or organ which contains the recombinant expression construct of claim 20.

23. A transgenic plant or plant cell capable of producing a biologically active urokinase, wherein said plant has a recombinant expression construct comprising a nucleotide sequence encoding a urokinase and a promoter that regulates expression of the nucleotide sequence in the transgenic plant or plant cell.

24. The transgenic plant or plant cell of claim 23, in which the promoter is an inducible promoter.

25. The transgenic plant or plant cell of claim 24, in which the inducible promoter is induced by mechanical gene activation.

26. The transgenic plant or plant cell of claim 25, in which the inducible promoter comprises the nucleotide sequence shown in Figure 3.

27. The transgenic plant or plant cell of claim 23, in which said transgenic plant or plant cell is a transgenic tobacco plant or tobacco cell.

28. The transgenic plant or plant cell of claim 23, wherein said urokinase is a human urokinase.

29. A leaf, stem, root, flower or seed of the transgenic plant of claim 28, which comprises a recombinant expression construct comprising a nucleotide sequence encoding a urokinase and a promoter that regulates expression of the nucleotide sequence.

## Patentansprüche

1. Verfahren zur Herstellung einer biologisch aktiven Urokinase in einer transgenen Pflanze, umfassend:
(a) Anzüchten (wachsen lassen) der transgenen Pflanze, die ein rekombinantes Expressionskonstrukt, enthaltend eine die Urokinase kodierende Nukleotidsequenz und einen Promotor, der die Expression der Nukleotidsequenz reguliert, aufweist, so dass die Urokinase von der transgenen Pflanze exprimiert wird; und
(b) Gewinnung der Urokinase aus einem Organ der transgenen Pflanze, wobei das Organ ein Blatt, Stamm, Wurzel, Blüte, Frucht oder Samen darstellt.

2. Verfahren nach Anspruch 1, worin der Promotor einen induzierbaren Promotor darstellt, und wobei das Verfahren zusätzlich zwischen den Schritten (a) und (b) den Schritt der Induzierung des induzierbaren Promotors vor oder nach der Ernte der transgenen Pflanze umfasst.

3. Verfahren nach Anspruch 2, worin der induzierbare Promotor durch mechanische Genaktivierung induziert wird.

4. Verfahren nach Anspruch 3, worin der induzierbare Promotor die in Fig. 3 dargestellte Nukleotidsequenz umfasst.

5. verfahren nach Anspruch 1, worin die transgene Pflanze eine transgene Tabakpflanze darstellt.

6. Verfahren nach Anspruch 1, worin die Urokinase eine menschliche Urokinase darstellt.

7. Verfahren nach Anspruch 6, worin die Urokinase die Aminosäuren 2 - 411 der in Fig. 2c dargestellten Sequenz umfasst.

8. Verfahren nach Anspruch 1, worin das Expressionskonstrukt die in Fig. 2a dargestellte Aminosäuresequenz kodiert.

9. Verfahren nach Anspruch 1, worin das Expressionskonstrukt die in Fig. 2b dargestellte Aminosäuresequenz kodiert.

10. Verfahren nach Anspruch 1, worin das Expressionskonstrukt die in Fig. 1a dargestellte Nukleotidsequenz enthält.

11. Verfahren nach Anspruch 10, worin das Expressionskonstrukt die in Fig. 1a dargestellte Nukleotidsequenz unter der Kontrolle des in Fig. 3 dargestellten Promotors enthält.

12. Verfahren nach Anspruch 1, worin das Expressionskonstrukt die in Fig. 1b dargestellte Nukleotidsequenz umfasst.

13. Verfahren nach Anspruch 12, worin das Expressionskonstrukt die in Fig. 1b dargestellte Nukleotidsequenz unter der Kontrolle des in Fig. 3 dargestellten Promotors umfasst,

14. Verfahren nach Anspruch 1, worin das Expressionskonstrukt die in Fig. 1c dargestellte Nukleotidsequenz umfasst.

15. Verfahren nach Anspruch 14, worin das Expressionskonstrukt die in Fig. 1c dargestellte Nukleotidsequenz unter der Kontrolle des in Fig. 3 dargestellten Promotors umfasst.

16. Rekombinantes Expressionskonstrukt, enthaltend eine für eine Urokinase kodierende Nukleotidsequenz und einen Promotor, der die Expression der Nukleotidsequenz in einer Pflanzenzelle reguliert.

17. Rekombinantes Expressionskonstrukt nach Anspruch 16, worin der Promotor einen induzierbaren Promotor darstellt.

18. Rekombinantes Expressionskonstrukt nach Anspruch 17, worin der induzierbare Promotor durch mechanische Genaktivierung induziert wird.

19. Rekombinantes Expressionskonstrukt nach Anspruch 18, worin der induzierbare Promotor die in Fig. 3 dargestellte Nukleotidsequenz umfasst.

20. Rekombinantes Expressionskonstrukt nach Anspruch 16, worin die Urokinase eine menschliche Urokinase darstellt.

21. Pflanzen-Transformationsvektor, enthaltend das rekombinante Expressionskonstrukt nach Anspruch 20.

22. Pflanzliche Zelle, Gewebe oder Organ, enthaltend das rekombinante Expressionskonstrukt nach Anspruch 20.

23. Transgene Pflanze oder Pflanzenzelle, die in der Lage ist, eine biologisch aktive Urokinase zu erzeugen, worin die Pflanze ein rekombinantes Expressionskonstrukt, enthaltend eine für eine Urokinase kodierende Nukleotidsequenz und einen Promotor, der die Expression der Nukleotidsequenz in der transgenen Pflanze oder Pflanzenzelle reguliert, aufweist.

24. Transgene Pflanze oder Pflanzenzelle nach Anspruch 23, worin der Promotor einen induzierbaren Promotor darstellt.

25. Transgene Pflanze oder Pflanzenzelle nach Anspruch 24, worin der induzierbare Promotor durch mechanische Genaktivierung induziert wird.

26. Transgene Pflanze oder Pflanzenzelle nach Anspruch 25, worin der induzierbare Promotor die in Fig. 3 dargestellte Nukleotidsequenz umfasst.

27. Transgene Pflanze oder Pflanzenzelle nach Anspruch 23, worin die transgene Pflanze oder Pflanzenzelle eine transgene Tabakpflanze oder Tabakpflanzenzelle darstellt.

28. Transgene Pflanze oder Pflanzenzelle nach Anspruch 23, worin die Urokinase eine menschliche Urokinase darstellt.

29. Blatt. Stamm, Wurzel, Blüte oder Samen der transgenen Pflanze nach Anspruch 28, die ein rekombinantes Expressionskonstrukt, enthaltend eine für eine Urokinase kodierende Nukleotidsequenz und einen Promotor, der die Expression der Nukleotidsequenez reguliert, aufweist.

## Revendications

1. Procédé pour la production d'une urokinase biologiquement active dans une plante transgénique, comprenant les étapes consistant :
(a) à cultiver la plante transgénique qui possède un produit d'assemblage d'expression recombinant comprenant une séquence de nucléotides cedant pour l'urokinase et un promoteur qui régule l'expression de la séquence de nucléotides de telle sorte que l'urokinase soit exprimée par la plante transgénique ; et
(b) à recueillir l'urokinase à partir d'un organe de la plante transgénique, l'organe étant une feuille, une tige, une racine, une fleur, un fruit ou une graine.

2. Procédé suivant la revendication 1, dans lequel le promoteur est un promoteur inductible, procédé qui comprend en outre, entre les étapes (a) et (b), l'étape d'induction du promoteur inductible avant ou après avoir récolté la plante transgénique.

3. Procédé suivant la revendication 2, dans lequel le promoteur inductible est induit par une activation mécanique de gène.

4. Procédé suivant la revendication 3, dans lequel le promoteur inductible comprend la séquence de nucléotides représentée sur la figure 3.

5. Procédé suivant la revendication 1, dans lequel la plante transgénique est un plant de tabac transgénique.

6. Procédé suivant la revendication 1, dans lequel l'urokinase est l'urokinase humaine.

7. Procédé suivant la revendication 6, dans lequel ladite urokinase comprend les aminoacides 2 à 411 de la séquence représentée sur la figure 2c.

8. Procédé suivant la revendication 1, dans lequel ledit produit d'assemblage d'expression code pour la séquence d'aminoacides représentée sur la figure 2a.

9. Procédé suivant la revendication 1, dans lequel ledit produit d'assemblage d'expression code pour la séquence d'aminoacides représentée sur la figure 2b.

10. Procédé suivant la revendication 1, dans lequel ledit produit d'assemblage d'expression comprend la séquence de nucléotides représentée sur la figure 1a.

11. Procédé suivant la revendication 10, dans lequel ledit produit d'expression comprend la séquence de nucléotides représentée sur la figure la sous le contrôle du promoteur représenté sur la figure 3.

12. Procédé suivant la revendication 1, dans lequel ledit produit d'assemblage d'expression comprend la séquence de nucléotides représentée sur la figure 1b.

13. Procédé suivant la revendication 12, dans lequel ledit précuit d'expression comprend la séquence de nucléotides représentée sur la figure 1b sous le contrôle du promoteur représenté sur la figure 3.

14. Procédé suivant la revendication 1, dans lequel ledit produit d'assemblage d'expression comprend la séquence de nucléotides représentée sur la figure 1c.

15. Précédé suivant la revendication 14, dans lequel ledit produit d'expression comprend la séquence de nucléotides représentée sur la figure 1c sous le contrôle du promoteur représenté sur la figure 3.

16. Produit d'assemblage d'expression recombinant comprenant une séquence de nucléotides codant pour une urokinase et un promoteur qui régule l'expression de la séquence de nucléotides dans une cellule végétale.

17. Produit d'assemblage d'expression recombinant suivant la revendication 16, dans lequel le promoteur est un promoteur inductible.

18. Produit d'assemblage d'expression recombinant suivant la revendication 17, dans lequel le promoteur inductible est induit par activation mécanique de gène.

19. Produit d'assemblage d'expression recombinant suivant la revendication 18, dans lequel le promoteur inductible comprend la séquence de nucléotides représentée sur la figure 3.

20. Produit d'assemblage d'expression recombinant suivant la revendication 16, dans lequel l'urokinase est une urokinase humaine.

21. Vecteur de transformation végétale comprenant le produit d'assemblage d'expression recombinant suivant la revendication 20.

22. Cellule végétale, tissu végétal ou organe végétal qui contient le produit d'assemblage d'expression recombinant suivant la revendication 20.

23. Plante transgénique ou cellule végétale transgénique capable de produire une urokinase biologiquement active, ladite plante possédant un produit d'assemblage d'expression recombinant comprenant une séquence de nucléotides codant pour une urokinase et un promoteur qui régule l'expression de la séquence de nucléotides dans la plante transgénique ou cellule végétale transgénique.

24. Plante transgénique ou cellule végétale transgénique suivant la revendication 23, dans laquelle le promoteur est un promoteur inductible .

25. plante transgénique ou cellule végétale transgénique suivant la revendication 24, dans laquelle le promoteur inductible est induit par activation mécanique de gène.

26. Plante transgénique ou cellule végétale transgénique suivant la revendication 25, dans laquelle le promoteur inductible comprend la séquence de nucléotides représentée sur la figure 3.

27. Plante transgénique ou cellule végétale transgénique suivant la revendication 23, ladite plante transgénique ou cellule végétale transgénique étant un plant de tabac transgénique ou une cellule de tabac transgénique.

28. Plante transgénique ou cellule végétale transgénique suivant la revendication 23, dans laquelle ladite urokinase est une urokinase humaine.

29. Feuille, tige, racine, fleur ou graine de la plante transgénique suivant la revendication 28, qui comprend un produit d'assemblage d'expression recombinant comprenant une séquence de nucléotides codant pour une urokinase et un promoteur qui régule l'expression de la séquence de nucléotides.
